(19) Europäisches Patentamt / European Patent Office / Office européen des brevets

(11) **EP 3 936 536 A1**

(12) **EUROPEAN PATENT APPLICATION**
published in accordance with Art. 153(4) EPC

(43) Date of publication:
**12.01.2022 Bulletin 2022/02**

(51) Int Cl.:
**C08F 20/06** (2006.01)   **A61F 13/15** (2006.01)
**A61F 13/49** (2006.01)   **A61F 13/53** (2006.01)

(21) Application number: 20769201.3

(22) Date of filing: **05.03.2020**

(86) International application number:
**PCT/JP2020/009475**

(87) International publication number:
**WO 2020/184387 (17.09.2020 Gazette 2020/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.03.2019  JP 2019042962**

(71) Applicant: **Sumitomo Seika Chemicals Co., Ltd.**
**Kako-gun, Hyogo 675-0145 (JP)**

(72) Inventor: **KAWAHARA Toru**
**Himeji-shi, Hyogo 672-8076 (JP)**

(74) Representative: **Nederlandsch Octrooibureau**
**P.O. Box 29720**
**2502 LS The Hague (NL)**

(54) **WATER ABSORBING RESIN PARTICLES AND METHOD FOR PRODUCING SAME, ABSORBENT BODY, AND ABSORBENT ARTICLE**

(57)   Disclosed is an absorbent article 100 including an absorber 10 containing water-absorbent resin particles 10a, in which the water-absorbent resin particles 10a have a maximum gel resistance measured by procedures (1) to (3) of 0.3 to 1.5 N, and a water absorption amount for physiological saline under a load of 4.14 kPa of 12 mL/g or more.

*Fig.1*

EP 3 936 536 A1

## Description

### Technical Field

The present invention relates to water-absorbent resin particles and a method for producing the same, an absorber, and an absorbent article.

### Background Art

In the related art, an absorber containing water-absorbent resin particles has been used in an absorbent article for absorbing a liquid (for example, urine) containing water as a main component. For example,

Patent Literature 1 below discloses water-absorbent resin particles having a particle diameter that is suitably used for absorbent articles such as diapers. In addition, Patent Literature 2 discloses a method of using a hydrogel-absorbent polymer having specific saline flow inducibility, performance under pressure, and the like as an effective absorbent member for housing a body fluid such as urine.

### Citation List

### Patent Literature

[Patent Literature 1] Japanese Unexamined Patent Publication No. H6-345819
[Patent Literature 2] Published Japanese Translation No. H9-510889 of the PCT International Publication

## Summary of Invention

### Technical Problem

In a case where a liquid provided to an absorbent article does not sufficiently permeate the absorbent article, there may be a problem of the excess liquid flowing on a surface of the absorbent article, resulting in its leakage to the outside of the absorbent article. Therefore, an absorbent article is required to cause a liquid to permeate it at a better permeation rate.

One aspect of the present invention is to provide water-absorbent resin particles that enable obtainment of an absorbent article having a better permeation rate, and a method for producing the water-absorbent resin particles. Another aspect of the present invention is to provide an absorber and an absorbent article which are formed using the water-absorbent resin particles.

### Solution to Problem

One aspect of the present invention provides water-absorbent resin particles, in which a maximum gel resistance measured by the following procedures (1) to (3) is 0.3 to 1.5 N, and a water absorption amount for physiological saline under a load of 4.14 kPa is 12 mL/g or more.

(1) A jig and a cylindrical container are prepared, the jig having a disk portion and a rod-shaped portion, the disk portion being 5 mm in thickness and having flat surfaces with a diameter of 2 cm on front and back sides, the rod-shaped portion having a circular cross-section with a diameter of 5 mm, one end of the rod-shaped portion being connected to a center of a first surface that is one of the flat surfaces of the disk portion, the cylindrical container having a bottom surface with an inner diameter of 4.6 cm, 58 g of physiological saline being placed in the cylindrical container. The jig and the cylindrical container are disposed so that a central axis in a height direction of the cylindrical container is located at a center of the disk portion.

(2) A swollen gel in which the jig is embedded is formed by putting 2.0 g of the water-absorbent resin particles into the cylindrical container in a state where the jig is immersed so that a second surface of the disk portion, the second surface being a flat surface on a side opposite to the first surface, is at a position 15 mm vertically below a water surface of the physiological saline.

(3) When the jig is raised vertically upward at a rate of 10 mm/min, a maximum value of a load applied to the jig is measured as the maximum gel resistance.

According to the above-described water-absorbent resin particles, it is possible to obtain an absorbent article having a better permeation rate.

Another aspect of the present invention provides an absorber containing the above-described water-absorbent resin particles.

Still another aspect of the present invention provides an absorbent article including the above-described absorber.

Still another aspect of the present invention provides a method for producing water-absorbent resin particles, the method including selecting water-absorbent resin particles based on a maximum gel resistance measured by the following procedures (1) to (3), and a water absorption amount for physiological saline under a load of 4.14 kPa.

(1) A jig and a cylindrical container are prepared, the jig having a disk portion and a rod-shaped portion, the disk portion being 5 mm in thickness and having flat surfaces with a diameter of 2 cm on front and back sides, the rod-shaped portion having a circular cross-section with a diameter of 5 mm, one end of the rod-shaped portion being connected to a center of a first surface that is one of the flat surfaces of the disk portion, the cylindrical container having a bottom surface with an inner diameter of 4.6 cm, 58 g of physiological saline being placed in the cylindrical container. The jig and the cylindrical container are disposed so that a central axis in a height direction of the cylindrical container is located at a center of the disk portion.

(2) A swollen gel in which the jig is embedded is formed by putting 2.0 g of the water-absorbent resin particles into the cylindrical container in a state where the jig is immersed so that a second surface of the disk portion, the second surface being a flat surface on a side opposite to the first surface, is at a position 15 mm vertically below a water surface of the physiological saline.

(3) When the jig is raised vertically upward at a rate of 10 mm/min, a maximum value of a load applied to the jig is measured as the maximum gel resistance.

According to the above-described method for producing water-absorbent resin particles, it is possible to obtain water-absorbent resin particles that enable obtainment of an absorbent article having a better permeation rate.

**Advantageous Effects of Invention**

According to one aspect of the present invention, it is possible to provide water-absorbent resin particles that enable obtainment of an absorbent article having a better permeation rate, and a method for producing the water-absorbent resin particles. Furthermore, according to another aspect of the present invention, it is possible to provide an absorber and an absorbent article which are formed using the water-absorbent resin particles. According to still another aspect of the present invention, it is possible to provide applications of resin particles, an absorber, and an absorbent article to absorption of a liquid.

**Brief Description of Drawings**

Fig. 1 is a cross-sectional view showing an example of an absorbent article.
Fig. 2 is a plan view showing an outline of a stirring blade used in Examples.
Fig. 3 is a schematic view showing a measurement device for a water absorption amount of water-absorbent resin particles under a load.
Fig. 4 is a schematic view showing a measurement device for a maximum gel resistance which was used in Examples.
Fig. 5 is a graph showing a transition of a load applied to a jig.

**Description of Embodiments**

Hereinafter, embodiments of the present invention will be described in detail. However, the present invention is not limited to the following embodiments, and can be variously modified and implemented within the scope of the gist thereof.

In the present specification, "acrylic" and "methacrylic" are collectively referred to as "(meth)acrylic." "Acrylate" and "methacrylate" are also referred to as "(meth)acrylate." "(Poly)" means both of a case where there is a prefix of "poly" and a case where there is no prefix thereof. Regarding numerical value ranges described in a stepwise manner in the present specification, an upper limit value or a lower limit value of a numerical value range in a certain step can be arbitrarily combined with an upper limit value or a lower limit value of a numerical value range in another step. In a numerical value range described in the present specification, an upper limit value or a lower limit value of the numerical value range may be replaced with a value shown in examples. The term "water-soluble" means that a solubility of 5% by mass or more is exhibited in water at 25°C. For materials exemplified in the present specification, one kind may be used alone, or two or more kinds may be used in combination. In a case where there are a plurality of substances corresponding to each of components in a composition, a content of each of the components in the composition means a total amount of the plurality of substances present in the composition unless otherwise specified. "Physiological saline" refers to an aqueous solution of 0.9% by mass sodium chloride.

In water-absorbent resin particles according to the present embodiment, a maximum gel resistance measured by the following procedures (1) to (3) is 0.3 to 1.5 N, and a water absorption amount for physiological saline under a load of 4.14 kPa is 12 mL/g or more.

(1) A jig and a cylindrical container are prepared, the jig having a disk portion and a rod-shaped portion, the disk portion being 5 mm in thickness and having flat surfaces with a diameter of 2 cm on front and back sides, the rod-shaped portion having a circular cross-section with a diameter of 5 mm, one end being connected to a center of a first surface that is one of the flat surfaces of the disk portion, the cylindrical container having a bottom surface with an inner diameter of 4.6 cm, 58 g of physiological saline being placed in the cylindrical container. The jig and the cylindrical container are disposed so that a central axis in a height direction of the cylindrical container is located at a center of the disk portion.

(2) A swollen gel in which the jig is embedded is formed by putting 2.0 g of the water-absorbent resin particles into the cylindrical container in a state where the jig is immersed so that a second surface of the disk portion, the second surface being a flat surface on a side opposite to the first surface, is at a position 15 mm vertically below a water surface of the physiological saline.

(3) When the jig is raised vertically upward at a rate of 10 mm/min, a maximum value of a load applied to the jig is measured as the maximum gel resistance.

According to water-absorbent resin particles, it is possible to obtain an absorbent article having a better permeation rate. The reason why such an effect is obtained is not clear, but the inventors of the present invention speculates as follows. However, the reason is not limited to the following contents. That is, in the water-absorbent resin particles according to the present embodiment, since a maximum gel resistance is 0.3 to 1.5 and a water absorption amount for physiological saline under a load of 4.14 kPa is 12 mL/g or more, swollen gel particles are easily peeled off even when they come into close contact with each other with absorbing liquid, and therefore a swollen gel having appropriate voids is formed. It is thought that the above-described effect can be obtained by appropriate voids, which are formed when a liquid is absorbed, functioning as a flow path inside the swollen gel.

A maximum gel resistance may be, for example, 1.5 N or less, 1.4 N or less, 1.3 N or less, 1.2 N or less, or 1.1 N or less, and may be for example, 0.3 N or more, 0.4 N or more, 0.5 N or more, 0.6 N or more, 0.7 N or more, 0.8 N or more, or 0.9 N or more, from the viewpoint that a better permeation rate is then easily obtained in an absorbent article. A maximum gel resistance may be, for example, 0.4 to 1.5 N, 0.5 to 1.5 N, 0.6 to 1.4 N, 0.7 to 1.3 N, 0.8 to 1.2 N, or 0.9 to 1.1 N, from the viewpoint that a better permeation rate is then easily obtained in an absorbent article. The maximum gel resistance may be a maximum gel resistance at room temperature (25°C $\pm$ 2°C).

A maximum gel resistance measured by the above-described procedures (1) to (3) can be specifically measured by a method described in Examples to be described later.

A water absorption amount for physiological saline under a load of 4.14 kPa of the water-absorbent resin particles according to the present embodiment is 12 mL/g or more, and it is preferably 15 mL/g or more, 18 mL/g or more, 20 mL/g or more, 22 mL/g or more, or 25 mL/g or more, from the viewpoint that a better permeation rate is then easily obtained in an absorbent article. A water absorption amount is preferably 40 mL/g or less, 35 mL/g or less, or 30 mL/g or less, from the viewpoint that excessive swelling is then easily inhibited in an absorbent article. From these viewpoints, a water absorption amount is preferably 12 to 40 mL/g, is more preferably 15 to 35 mL/g, and is even more preferably 22 to 35 mL/g. The water absorption amount may be a water absorption amount at room temperature (25°C $\pm$ 2°C). The water absorption amount can be measured by a method described in Examples to be described later.

It is sufficient for the water-absorbent resin particles of the present embodiment to be able to retain water, and a liquid that is an absorption target liquid can include water. The water-absorbent resin particles according to the present embodiment are better in absorbability of body fluids such as urine, sweat, and blood (for example, menstrual blood). The water-absorbent resin particles according to the present embodiment can be used as a constituent component of an absorber according to the present embodiment.

A water retention amount (water retention amount under no pressure) for physiological saline of the water-absorbent resin particles according to the present embodiment is preferably within the following range. A water retention amount is preferably 10 g/g or more, 15 g/g or more, 20 g/g or more, 25 g/g or more, or 30 g/g or more, from the viewpoint that a better permeation rate is then easily obtained in an absorbent article. A water retention amount is preferably 80 g/g or less, 75 g/g or less, 70 g/g or less, 65 g/g or less, 60 g/g or less, 55 g/g or less, 50 g/g or less, 48 g/g or less, 45 g/g or less, 42 g/g or less, 40 g/g or less, or 35 g/g or less, from the viewpoint that a better permeation rate is then easily obtained in an absorbent article. From these viewpoints, a water retention amount is preferably 10 to 80 g/g. The water retention amount may be a water retention amount at room temperature (25°C ± 2°C). The water retention amount can be measured by a method described in Examples to be described later.

A water absorption amount for physiological saline under no pressure of the water-absorbent resin particles according to the present embodiment may be 45.0 g/g or more, 48.0 g/g or more, 50.0 g/g or more, 51.0 g/g or more, or 55.0 g/g or more, and may be 70 g/g or less, 65 g/g or less, 63 g/g or less, or 60 g/g or less, from the viewpoint that a better permeation rate is then easily obtained in an absorbent article. The water absorption amount under no pressure may be a water absorption amount at room temperature (25°C ± 2°C). The water absorption amount under no pressure can be measured by a method described in Examples to be described later.

Examples of shapes of the water-absorbent resin particles according to the present embodiment include a substantially spherical shape, a crushed shape, a granular shape, and the like. A median particle diameter of the water-absorbent resin particles (water-absorbent resin particles before absorbing water) according to the present embodiment is preferably within the following range. A median particle diameter is preferably 250 μm or more, 280 μm or more, 300 μm or more, 310 μm or more, 320 μm or more, 330 μm or more, 340 μm or more, 350 μm or more, or 360 μm or more, from the viewpoint that then, gel blocking is avoided, and a favorable permeation rate of an absorbent article is easily maintained. A median particle diameter is preferably 600 μm or less, 550 μm or less, 500 μm or less, 450 μm or less, 400 μm or less, 380 μm or less, or 370 μm or less, from the viewpoint that a soft tactile sensation of an absorbent article is then easily maintained. From these viewpoints, a median particle diameter is preferably 250 to 600 μm. The water-absorbent resin particles according to the present embodiment may have a desired particle diameter distribution at a timing obtained in a production method to be described later, but their particle diameter distribution may be adjusted by performing operations such as adjustment of a particle diameter through classification with a sieve.

The water-absorbent resin particles according to the present embodiment can contain, for example, a cross-linking polymer, which are obtained by polymerization of monomers including ethylenically unsaturated monomers, as polymer particles. That is, the water-absorbent resin particles according to the present embodiment can have a structural unit derived from ethylenically unsaturated monomers. As the ethylenically unsaturated monomer, a water-soluble ethylenically unsaturated monomer can be used. Examples of methods of polymerization include a reverse phase suspension polymerization method, an aqueous solution polymerization method, a bulk polymerization method, and a precipitation polymerization method. Among them, the reverse phase suspension polymerization method or the aqueous solution polymerization method is preferable from the viewpoints of facilitating securement of favorable water-absorbent characteristics (such as a water retention amount) of the obtained water-absorbent resin particles and control of a polymerization reaction. Hereinbelow, a method for polymerizing ethylenically unsaturated monomers will be described with the reverse phase suspension polymerization method as an example.

An ethylenically unsaturated monomer is preferably water-soluble. Examples thereof include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where an ethylenically unsaturated monomer has an amino group, the amino group may be quaternarized. The ethylenically unsaturated monomer may be used alone or in a combination of two or more kinds thereof. A functional group, such as a carboxyl group and an amino group, of the monomer, may function as a crosslinkable functional group in a surface cross-linking step to be described later.

Among them, from the viewpoint of high industrial availability, the ethylenically unsaturated monomer preferably includes at least one compound selected from the group consisting of (meth)acrylic acid and a salt thereof, acrylamide, methacrylamide, and N,N-dimethyl acrylamide, and more preferably includes at least one compound selected from the group consisting of (meth)acrylic acid and a salt thereof, and acrylamide. The ethylenically unsaturated monomer more preferably includes at least one compound selected from the group consisting of (meth)acrylic acid and a salt thereof from the viewpoint of further enhancing water-absorbent characteristics (such as a water retention amount). That is, the water-absorbent resin particles preferably have a structural unit derived from at least one selected from the group consisting of (meth)acrylic acid and a salt thereof.

For the monomer for obtaining the water-absorbent resin particles, a monomer other than the above-described ethylenically unsaturated monomers may be used. Such a monomer can be used by, for example, being mixed with an aqueous solution containing the above-described ethylenically unsaturated monomers. A use amount of the ethylenically unsaturated monomer may be 70 to 100 mol%, 80 to 100 mol%, 90 to 100 mol%, 95 to 100 mol%, or 100 mol%, with respect to a total amount of the monomers (the total amount of the monomers for obtaining the water-absorbent resin particles, for example, a total amount of monomers that gives a structural unit of a cross-linking polymer, the same shall apply hereinafter). Among them, a proportion of (meth)acrylic acid and a salt thereof may be 70 to 100 mol%, 80 to 100 mol%, 90 to 100 mol%, 95 to 100 mol%, or 100 mol%, with respect to a total amount of monomers. The "proportion of (meth)acrylic acid and a salt thereof" means a proportion of a total amount of (meth)acrylic acid and a salt thereof.

According to the present embodiment, as an example of the water-absorbent resin particles, it is possible to provide water-absorbent resin particles which contain a cross-linking polymer having a structural unit derived from an ethylenically unsaturated monomer, in which the ethylenically unsaturated monomer includes at least one compound selected from the group consisting of (meth)acrylic acid and a salt thereof, and a proportion of (meth)acrylic acid and a salt thereof is 70 to 100 mol% with respect to a total amount of monomers for obtaining the water-absorbent resin particles.

Usually, the ethylenically unsaturated monomers are suitably used in a form of an aqueous solution. A concentration of the ethylenically unsaturated monomer in an aqueous solution containing the ethylenically unsaturated monomer (hereinafter, simply referred to as a "monomer aqueous solution") is preferably 20% by mass or more and a saturated concentration or less, is more preferably 25% to 70% by mass, and is even more preferably 30% to 55% by mass. Examples of water to be used in an aqueous solution include tap water, distilled water, and ion-exchanged water.

In a case where ethylenically unsaturated monomers have an acid group, a monomer aqueous solution may be used after neutralizing this acid group with an alkaline neutralizing agent. A degree of neutralization of the ethylenically unsaturated monomer by the alkaline neutralizing agent is preferably 10 to 100 mol%, more preferably 50 to 90 mol%, and even more preferably 60 to 80 mol% of the acid group in the ethylenically unsaturated monomer, from a viewpoint of increasing an osmotic pressure of the obtained water-absorbent resin particles, and further enhancing water-absorbent characteristics (water retention amount and the like). Examples of alkaline neutralizing agents include alkali metal salts such as sodium hydroxide, sodium carbonate, sodium hydrogen carbonate, potassium hydroxide, and potassium carbonate; ammonia; and the like. The alkaline neutralizing agent may be used alone or in combination of two or more kinds thereof. The alkaline neutralizing agent may be used in a form of an aqueous solution to simplify a neutralizing operation. Neutralization of the acid groups in the ethylenically unsaturated monomers can be performed by, for example, adding an aqueous solution of sodium hydroxide, potassium hydroxide, or the like dropwise to the above-described monomer aqueous solution and mixing them.

In a reverse phase suspension polymerization method, a monomer aqueous solution is dispersed in a hydrocarbon dispersion medium in the presence of a surfactant, and polymerization of the ethylenically unsaturated monomer can be performed using a radical polymerization initiator or the like. As the radical polymerization initiator, a water-soluble radical polymerization initiator can be used.

Examples of surfactants include nonionic surfactants and anionic surfactants. Examples of nonionic surfactants include sorbitan fatty acid esters, polyglycerin fatty acid esters, sucrose fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkylphenyl ethers, polyoxyethylene castor oil, polyoxyethylene hydrogenated castor oil, alkylallyl formaldehyde condensed polyoxyethylene ethers, polyoxyethylene polyoxypropylene block copolymers, polyoxyethylene polyoxypropyl alkyl ethers, and polyethylene glycol fatty acid esters. Examples of anionic surfactants include fatty acid salts, alkylbenzene sulfonate, alkylmethyl taurate, polyoxyethylene alkylphenyl ether sulfuric acid ester salts, polyoxyethylene alkyl ether sulfonic acid salts, phosphoric acid esters of polyoxyethylene alkyl ethers, and phosphoric acid esters of polyoxyethylene alkyl allyl ethers. The surfactant may be used alone or in combination of two or more kinds thereof.

The surfactant preferably includes at least one compound selected from the group consisting of sorbitan fatty acid esters, polyglycerin fatty acid esters, and sucrose fatty acid esters, from the viewpoints that then, a state of a W/O type reverse-phase suspension becomes favorable, water-absorbent resin particles having a suitable particle diameter are easily obtained, and industrial availability becomes high. The surfactant preferably includes sucrose fatty acid ester, and more preferably includes sucrose stearic acid ester, from a viewpoint of easily obtaining an appropriate particle diameter distribution of the water-absorbent resin particles, and from a viewpoint of easily improving water-absorbent characteristics (such as a water retention amount) of the water-absorbent resin particles and performances of an absorbent article formed using the same.

A use amount of the surfactant is preferably 0.05 to 10 parts by mass, is more preferably 0.08 to 5 parts by mass, and is even more preferably 0.1 to 3 parts by mass, with respect to 100 parts by mass of the monomer aqueous solution, from the viewpoint that a sufficient effect is obtained within these use amounts, and these amounts are economic.

In the reverse phase suspension polymerization, a polymer-based dispersant may be used in combination with the above-mentioned surfactant. Examples of polymer-based dispersants include maleic anhydride-modified polyethylene, maleic anhydride-modified polypropylene, a maleic anhydride-modified ethylene-propylene copolymer, a maleic anhydride-modified EPDM (ethylene propylene diene terpolymer), maleic anhydride-modified polybutadiene, a maleic anhydride-ethylene copolymer, a maleic anhydride-propylene copolymer, a maleic anhydride-ethylene-propylene copolymer, a maleic anhydride-butadiene copolymer, polyethylene, polypropylene, an ethylene-propylene copolymer, oxidized polyethylene, oxidized polypropylene, an oxidized ethylene-propylene copolymer, an ethylene-acrylic acid copolymer, ethyl cellulose, ethyl hydroxyethyl cellulose, and the like. The polymer-based dispersant may be used alone or in combination of two or more kinds thereof. From the viewpoint of better dispersion stability of monomers, the polymer-based dispersant is preferably at least one selected from the group consisting of maleic anhydride-modified polyethylene, maleic anhydride-modified polypropylene, a maleic anhydride-modified ethylene-propylene copolymer, a maleic anhydride-ethylene copolymer, a maleic anhydride-propylene copolymer, a maleic anhydride-ethylene-propylene copolymer, polyethylene, polypropylene, an ethylene-propylene copolymer, oxidized polyethylene, oxidized polypropylene, and an oxidized ethylene-propylene copolymer.

A use amount of the polymer-based dispersant is preferably 0.05 to 10 parts by mass, is more preferably 0.08 to 5 parts by mass, and is even more preferably 0.1 to 3 parts by mass, with respect to 100 parts by mass of the monomer aqueous solution, from the viewpoint that a sufficient effect is obtained within these use amounts, and these amounts are economic.

The hydrocarbon dispersion medium may include at least one compound selected from the group consisting of a chained aliphatic hydrocarbon having 6 to 8 carbon atoms and an alicyclic hydrocarbon having 6 to 8 carbon atoms. Examples of hydrocarbon dispersion media include chained aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. The hydrocarbon dispersion medium may be used alone or in combination of two or more kinds thereof.

The hydrocarbon dispersion medium may include at least one selected from the group consisting of n-heptane and cyclohexane from the viewpoints of high industrial availability and stable qualities. Furthermore, from the same viewpoints, as a mixture of the hydrocarbon dispersion media, for example, a commercially available Exxsol Heptane (manufactured by ExxonMobil Chemical: containing n-heptane and 75% to 85% of hydrocarbons of isomers thereof) may be used.

A use amount of the hydrocarbon dispersion medium is preferably 30 to 1,000 parts by mass, is more preferably 40 to 500 parts by mass, and is even more preferably 50 to 400 parts by mass, with respect to 100 parts by mass of the monomer aqueous solution, from the viewpoint that polymerization heat is then appropriately removed, and thereby a polymerization temperature is easily controlled. In a case where a use amount of the hydrocarbon dispersion medium is 30 parts by mass or more, a polymerization temperature tends to be easily controlled. In a case where a use amount of the hydrocarbon dispersion medium is 1,000 parts by mass or less, productivity of polymerization tends to be improved, which is economical.

A radical polymerization initiator is preferably water-soluble. Examples thereof include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis[2-(2-imidazolin-2-yl)propane] dihydrochloride, 2,2'-azobis{2-[1-(2-hydroxyethyl)-2-imidazolin-2-yl]propane} dihydrochloride, 2,2'-azobis{2-methyl-N-[1,1 -bis(hydroxymethyl)-2-hydroxyethyl]propi onamide}, 2,2'-azobis[2-methyl-N-(2-hydroxyethyl)-propionamide], and 4,4'-azobis(4-cyanovaleric acid). The radical polymerization initiator may be used alone or in combination of two or more kinds thereof. The radical polymerization initiator is preferably at least one selected from the group consisting of potassium persulfate, ammonium persulfate, sodium persulfate, 2,2'-azobis(2-amidinopropane) dihydrochloride, 2,2'-azobis[2-(2-imidazolin-2-yl)propane] dihydrochloride, and 2,2'-azobis{2-[1-(2-hydroxyethyl)-2-imidazolin-2-yl]propane} dihydrochloride.

A use amount of the radical polymerization initiator may be 0.05 to 10 mmol with respect to 1 mole of the ethylenically unsaturated monomer. A case in which a use amount of the radical polymerization initiator is 0.05 mmol or more is efficient, because then a polymerization reaction is not required to be performed for a long period of time. In a case where a use amount of the radical polymerization initiator is 10 mmol or less, it is easy to inhibit occurrence of a rapid polymerization reaction.

The radical polymerization initiator can also be used as a redox polymerization initiator when it is used in combination with a reducing agent such as sodium sulfite, sodium hydrogen sulfite, ferrous sulfate, and L-ascorbic acid.

In a polymerization reaction, a monomer aqueous solution used for the polymerization may contain a chain transfer agent. Examples of chain transfer agents include hypophosphites, thiols, thiolic acids, secondary alcohols, and amines.

The monomer aqueous solution used for the polymerization may contain a thickener to control a particle diameter of the water-absorbent resin particles. Examples of thickeners include hydroxyethyl cellulose, hydroxypropyl cellulose, methyl cellulose, carboxymethyl cellulose, polyethylene glycol, polyacrylamide, polyethyleneimine, dextrin, sodium alginate, polyvinyl alcohol, polyvinylpyrrolidone, polyethylene oxide, and the like. In a case where rotation speeds in the polymerization are the same, a median particle diameter of particles to be obtained is likely to become large as a viscosity of the monomer aqueous solution becomes high.

Cross-linking by self-cross-linking may occur during polymerization, but cross-linking may be performed by using an internal cross-linking agent. By using the internal cross-linking agent, it is easy to control water-absorbent characteristics (such as a water retention amount) of the water-absorbent resin particles. The internal cross-linking agent is generally added to a reaction solution in the polymerization reaction. Examples of internal cross-linking agents include di- or tri(meth)acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the polyols with unsaturated acids (such as maleic acid and fumaric acid); bis(meth)acrylamides such as N,N'-methylenebis(meth)acrylamide; di- or tri(meth)acrylic acid esters obtained by reacting a polyepoxide with (meth)acrylic acid; carbamyl di(meth)acrylate esters obtained by reacting a polyisocyanate (such as tolylene diisocyanate and hexamethylene diisocyanate) with hydroxyethyl (meth)acrylate; compounds having two or more polymerizable unsaturated groups, such as allylated starch, allylated cellulose, diallyl phthalate, N,N',N"-triallyl isocyanate, and divinylbenzene; polyglycidyl compounds such as (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromohydrin, and $\alpha$-methyl epichlorohydrin; compounds having two or more reactive functional groups, such as isocyanate compounds (such as 2,4-tolylene diisocyanate and hexamethylene diisocyanate); and the like. The internal cross-linking agent may be used alone or in combination of two or more kinds thereof. The internal cross-linking agent is preferably a polyglycidyl compound, is more preferably a diglycidyl ether compound, and is even more preferably at least one selected from the group consisting of (poly)ethylene glycol diglycidyl ether, (poly)propylene glycol diglycidyl ether, and (poly)glycerin diglycidyl ether.

A use amount of the internal cross-linking agent is preferably 30 mmol or less, more preferably 0.01 to 10 mmol, even more preferably 0.012 to 5 mmol, particularly preferably 0.015 to 1 mmol, extremely preferably 0.02 to 0.1 mmol, and extraordinarily preferably 0.025 to 0.06 mmol per 1 mole of the ethylenically unsaturated monomer, from the viewpoints of easily obtaining a better permeation rate in an absorbent article, and inhibiting water-soluble properties by appropriately cross-linking the obtained polymer and thereby easily obtaining a sufficient water absorption amount.

The reverse phase suspension polymerization can be performed in a water-in-oil system by heating, under stirring, ethylenically unsaturated monomers, a radical polymerization initiator, a surfactant, a polymer-based dispersant, a hydrocarbon dispersion medium, and the like (and furthermore, an internal cross-linking agent as necessary) in a state where they are mixed.

When performing the reverse phase suspension polymerization, a monomer aqueous solution which contains ethylenically unsaturated monomers is dispersed in a hydrocarbon dispersion medium in the presence of a surfactant (and if necessary, a polymer-based dispersant). In this case, a timing of adding the surfactant, the polymer-based dispersant, or the like before the start of the polymerization reaction may be either before or after the addition of the monomer aqueous solution.

Among them, it is preferable to carry out the polymerization after dispersing the monomer aqueous solution in the hydrocarbon dispersion medium in which the polymer-based dispersant has been dispersed, and then further dispersing the surfactant in the hydrocarbon dispersion medium, from the viewpoint that an amount of the hydrocarbon dispersion medium remaining in the obtained water-absorbent resin can then be easily reduced.

The reverse phase suspension polymerization can be carried out in one stage or in multiple stages of two or more stages. The reverse phase suspension polymerization is preferably carried out in two or three stages from the viewpoint of increasing productivity.

In a case where reverse phase suspension polymerization is carried out in multiple stages of two or more stages, it is sufficient for stages after a second stage of reverse phase suspension polymerization to be carried out in the same manner as in a first stage of reverse phase suspension polymerization by adding ethylenically unsaturated monomers to a reaction mixture obtained in the first stage of polymerization reaction and mixing them, after performing the first stage of reverse phase suspension polymerization. In reverse phase suspension polymerization in each stage after the second stage, reverse phase suspension polymerization is preferably carried out by adding, in addition to ethylenically unsaturated monomers, the above-mentioned radical polymerization initiator and/or internal cross-linking agent within a range of molar ratios of the respective components to the ethylenically unsaturated monomers, based on an amount of ethylenically unsaturated monomers added during reverse phase suspension polymerization in each stage after the second stage. If necessary, the internal cross-linking agent may be used in reverse phase suspension polymerization in each stage after the second stage. In a case where the internal cross-linking agent is used, it is preferable to carry out reverse phase suspension polymerization by adding the internal cross-linking agent within a range of molar ratios of the respective components to the ethylenically unsaturated monomers based on an amount of ethylenically unsaturated monomers provided in each stage.

A temperature for the polymerization reaction varies depending on radical polymerization initiators used, but it is preferably 20°C to 150°C, and is more preferably 40°C to 120°C, from the viewpoint that the polymerization is then promptly performed, which shortens a polymerization time, and thereby economic efficiency increases, and that polymerization heat is then easily removed, and thereby the reaction is smoothly performed. A reaction time is generally 0.5 to 4 hours. Completion of the polymerization reaction can be confirmed from, for example, stop of temperature rising in the reaction system. Accordingly, a polymer of ethylenically unsaturated monomers is generally obtained in a state of a hydrogel.

After the polymerization, cross-linking may be carried out by adding a post-polymerization cross-linking agent to the obtained hydrogel-like polymer and heating them. By performing post-polymerization cross-linking, a degree of cross-linking of the hydrogel-like polymer can be increased, and water-absorbent characteristics (such as a water retention amount) can be further improved.

Examples of post-polymerization cross-linking agents include polyols such as ethylene glycol, propylene glycol, 1,4-butanediol, trimethylolpropane, glycerin, polyoxyethylene glycol, polyoxypropylene glycol, and polyglycerin; compounds having two or more epoxy groups, such as (poly)ethylene glycol diglycidyl ether, (poly)propylene glycol diglycidyl ether, and (poly)glycerin diglycidyl ether; haloepoxy compounds such as epichlorohydrin, epibromohydrin, and $\alpha$-methyl epichlorohydrin; compounds having two or more isocyanate groups such as 2,4-tolylene diisocyanate and hexamethylene diisocyanate; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide. Among them, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are preferable. The cross-linking agent may be used alone or in combination of two or more kinds thereof.

An amount of the post-polymerization cross-linking agent is preferably 30 mmol or less, more preferably 10 mmol or less, even more preferably 0.01 to 5 mmol, particularly preferably 0.012 to 1 mmol, extremely preferably 0.015 to 0.1 mmol, and extraordinarily preferably 0.02 to 0.05 mmol, per 1 mole of the ethylenically unsaturated monomer, from a viewpoint of easily obtaining suitable water-absorbent characteristics (such as a water retention amount).

It is sufficient for a timing for adding the post-polymerization cross-linking agent to be after polymerization of ethylenically unsaturated monomers used for the polymerization. In a case of multi-stage polymerization, the cross-linking agent is preferably added after the multi-stage polymerization. From the viewpoint of a water content (to be described later), it is preferable to add the post-polymerization cross-linking agent within a region of [water content immediately after polymerization ± 3% by mass], in consideration of heat generation during and after polymerization, retention due to step delay, system opening when a cross-linking agent is added, and fluctuation in moisture content due to addition of water associated with addition of a cross-linking agent.

Subsequently, the polymer particles (for example, polymer particles having a structural unit derived from an ethylenically unsaturated monomer) are obtained by drying in order to remove moisture from the obtained hydrogel-like polymer. Examples of drying methods include a method (a) in which a hydrogel-like polymer in a state of being dispersed in a hydrocarbon dispersion medium is subjected to azeotropic distillation by heating from the outside, and the hydrocarbon dispersion medium is refluxed to remove moisture; a method (b) in which a hydrogel-like polymer is taken out by decantation and dried under reduced pressure; and a method (c) in which a hydrogel-like polymer is separated by filtration with a filter and dried under reduced pressure. Among them, the method (a) is preferably used for its simplicity in production steps.

It is possible to adjust a particle diameter of the water-absorbent resin particles by adjusting a rotation speed of a stirrer during the polymerization reaction or by adding a flocculating agent to the system after the polymerization reaction or at an initial time of drying. A particle diameter of the obtained water-absorbent resin particle can be increased by adding the flocculating agent. As the flocculating agent, an inorganic flocculating agent can be used. Examples of inorganic flocculating agents (for example, powdery inorganic flocculating agents) include silica, zeolite, bentonite, aluminum oxide, talc, titanium dioxide, kaolin, clay, and hydrotalcite. The flocculating agent is preferably at least one selected from the group consisting of silica, aluminum oxide, talc, and kaolin from the viewpoint of a better flocculation effect.

In the reverse phase suspension polymerization, a method of adding the flocculating agent is preferably a method in which a flocculating agent is dispersed in a hydrocarbon dispersion medium of the same kind as that used in the polymerization, or water in advance, and then the mixture is mixed into a hydrocarbon dispersion medium containing a hydrogel-like polymer under stirring.

An amount of the flocculating agent added is preferably 0.001 to 1 part by mass, is more preferably 0.005 to 0.5 parts by mass, and is even more preferably 0.01 to 0.2 parts by mass, with respect to 100 parts by mass of ethylenically unsaturated monomers used in the polymerization. By setting an amount of the flocculating agent added to be within the above range, it is easy to obtain water-absorbent resin particles having a desired particle diameter distribution.

In the production of the water-absorbent resin particles, it is preferable to perform surface cross-linking of a surface portion (surface and in the vicinity of surface) of a hydrogel-like polymer using a surface cross-linking agent in a drying step (moisture removing step) or any subsequent steps. By performing surface cross-linking, a maximum gel resistance, water-absorbent characteristics (such as a water retention amount), and the like of the water-absorbent resin particles are easily controlled. The surface cross-linking is preferably performed at a timing when the hydrogel-like polymer has a specific water content. A timing of the surface cross-linking is preferably a time point at which a water content of the hydrogel-like polymer is 5% to 50% by mass, is more preferably a time point at which a water content thereof is 10% to 40% by mass, and is even more preferably a time point at which a water content thereof is 15% to 35% by mass. A water content (% by mass) of the hydrogel-like polymer is calculated by the following formula.

$$Water\ content = [Ww/(Ww + Ws)] \times 100$$

Ww: A moisture amount of a hydrogel-like polymer obtained by adding a moisture amount used, as desired, upon mixing a flocculating agent, a surface cross-linking agent, and the like to an amount obtained by subtracting a moisture amount extracted to the outside of the system by the drying step from a moisture amount contained in a monomer aqueous solution before polymerization in the all polymerization steps.

Ws: A solid fraction calculated from an amount of materials introduced, such as ethylenically unsaturated monomers, a cross-linking agent, and an initiator, each of which constitutes the hydrogel-like polymer.

Examples of surface cross-linking agents include compounds having two or more reactive functional groups. Examples of surface cross-linking agents include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and $\alpha$-methyl epichlorohydrin; isocyanate compounds such as 2,4-tolylene diisocyanate and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; hydroxyalkylamide compounds such as bis[N,N-di($\beta$-hydroxyethyl)]adipamide; and the like. The surface cross-linking agent may be used alone or in combination of two or more kinds thereof. The surface cross-linking agent is preferably a polyglycidyl compound, and is more preferably at least one selected from the group consisting of (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether.

A use amount of the surface cross-linking agent is preferably 0.01 to 20 mmol, more preferably 0.05 to 10 mmol, even more preferably 0.1 to 5 mmol, particularly preferably 0.15 to 1 mmol, and extremely preferably 0.2 to 0.5 mmol, per 1 mole of the ethylenically unsaturated monomer used for polymerization, from a viewpoint of easily obtaining suitable water-absorbent characteristics (such as a water retention amount).

After the surface cross-linking, it is possible to obtain polymer particles, which are a surface-cross-linked dried product, by distilling off water and the hydrocarbon dispersion medium by a known method, by drying under heating and reduced pressure, and the like.

The polymerization reaction can be carried out using various stirrers having a stirring blade. As the stirring blade, it is possible to use a flat plate blade, a lattice blade, a paddle blade, a propeller blade, an anchor blade, a turbine blade, a Pfaudler blade, a ribbon blade, a full zone blade, a max blend blade, and the like. A flat plate blade has a shaft (stirring shaft) and a flat plate portion (stirring portion) disposed around the shaft. Furthermore, the flat plate portion may have a slit and the like. In a case where the flat plate blade is used as the stirring blade, it is easy to uniformly carry out the cross-linking reaction in polymer particles, and it is easy to control a maximum gel resistance within a range suitable for the invention of the present application while maintaining water-absorbent characteristics such as a water retention amount.

In addition to the polymer particles, the water-absorbent resin particles according to the present embodiment can further contain, for example, various additional such as gel stabilizers, metal chelating agents (ethylenediaminetetraacetic acid and its salts, diethylenetriaminepentaacetic acid and its salts, for example, diethylenetriaminepentaacetic acid pentasodium, and the like), and fluidity improvers (lubricants). The additional components may be disposed inside the polymer particles, on a surface of the polymer particles, or both of the inside and on the surface thereof.

The water-absorbent resin particles may contain a plurality of inorganic particles disposed on the surface of the polymer particles. The inorganic particles can be disposed on the surface of the polymer particles by, for example, mixing the polymer particles and the inorganic particles. These inorganic particles may be silica particles such as amorphous silica.

In a case where the water-absorbent resin particles include inorganic particles disposed on the surface of the polymer particles, a content of the inorganic particles may be within the following range based on a total mass of the polymer particles. A content of the inorganic particles may be 0.05% by mass or more, 0.1% by mass or more, 0.15% by mass or more, or 0.2% by mass or more. A content of the inorganic particles may be 5.0% by mass or less, 3.0% by mass or less, 1.0% by mass or less, or 0.5% by mass or less.

The inorganic particles referred to herein generally have a minute size as compared with a size of the polymer particles. For example, an average particle diameter of the inorganic particles may be 0.1 to 50 $\mu$m, 0.5 to 30 $\mu$m, or 1 to 20 $\mu$m. The average particle diameter can be measured by a pore electric resistance method or a laser diffraction/scattering method depending on the characteristics of the particles.

An absorber according to the present embodiment contains the water-absorbent resin particles according to the present embodiment. The absorber according to the present embodiment may contain a fibrous substance, and it is, for example, a mixture containing the water-absorbent resin particles and the fibrous substance. The configuration of the absorber may be, for example, a configuration in which water-absorbent resin particles and the fibrous substances are uniformly mixed, a configuration in which water-absorbent resin particles are held between fibrous substances formed in a sheet shape or a layer shape, or another configuration.

Examples of fibrous substances include finely pulverized wood pulp; cotton; cotton linter; rayon; cellulose-based fibers such as cellulose acetate; synthetic fibers such as polyamides, polyesters, and polyolefins; a mixture of these fibers; and the like. The fibrous substance may be used alone or in combination of two or more kinds thereof. As the fibrous substance, hydrophilic fibers can be used.

Fibers may be adhered to each other by adding an adhesive binder to the fibrous substance in order to enhance shape retention properties before or during use of the absorber. Examples of adhesive binders include heat-sealing synthetic fibers, hot melt adhesives, and adhesive emulsions. The adhesive binder may be used alone or in combination of two or more kinds thereof.

Examples of heat-sealing synthetic fibers include full-melt binders such as polyethylene, polypropylene, and an ethylene-propylene copolymer; and partial-melt binders formed of polypropylene and polyethylene in a side-by-side or core-and-sheath configuration. In the above-mentioned partial-melt binders, only a polyethylene portion can be thermal-bonded.

Examples of hot melt adhesives include a mixture of a base polymer such as an ethylene-vinyl acetate copolymer, a styrene-isoprene-styrene block copolymer, a styrene-butadiene-styrene block copolymer, a styrene-ethylene-butylene-styrene block copolymer, a styrene-ethylene-propylene-styrene block copolymer, and an amorphous polypropylene with a viscosity imparting agent, a plasticizer, an antioxidant, or the like.

Examples of adhesive emulsions include polymers of at least one monomer selected from the group consisting of methyl methacrylate, styrene, acrylonitrile, 2-ethylhexyl acrylate, butyl acrylate, butadiene, ethylene, and vinyl acetate.

The absorber according to the present embodiment may contain inorganic powders (for example, amorphous silica), deodorants, antibacterial agents, pigments, dyes, fragrances, pressure sensitive adhesives, and the like. In a case where the water-absorbent resin particles contain inorganic particles, the absorber may contain an inorganic powder in addition to the inorganic particles in the water-absorbent resin particles.

A shape of the absorber according to the present embodiment may be, for example, a sheet shape. A thickness of the absorber (for example, a thickness of a sheet-shaped absorber) may be 0.1 to 20 mm or 0.3 to 15 mm.

A content of the water-absorbent resin particles in the absorber may be 2% to 100% by mass, 10% to 80% by mass, or 20% to 60% by mass, with respect to a total of the water-absorbent resin particles and the fibrous substance, from a viewpoint of easily obtaining sufficient water-absorbent characteristics.

A content of the water-absorbent resin particles in the absorber is preferably 100 to 1,000 g, is more preferably 150 to 800 g, and is even more preferably 200 to 700 g, per 1 $m^2$ of the absorber from the viewpoint of easily obtaining sufficient water-absorbent characteristics. A content of the fibrous substance in the absorber is preferably 50 to 800 g, is more preferably 100 to 600 g, and is even more preferably 150 to 500 g, per 1 $m^2$ of the absorber from the viewpoint of easily obtaining sufficient water-absorbent characteristics.

An absorbent article according to the present embodiment includes the absorber according to the present embodiment. Examples of other constituent members of the absorbent article according to the present embodiment include a core wrap that retains the shape of the absorber and also prevents the constituent members of the absorber from falling off or flowing; a liquid permeable sheet disposed on the outermost part on a side from which an absorption target liquid is infiltrated; a liquid impermeable sheet disposed on the outermost part on a side opposite to the side from which the absorption target liquid is infiltrated; and the like. Examples of the absorbent article include diapers (for example, paper diapers), toilet training pants, incontinence pads, hygiene products (sanitary napkins, tampons, and the like), sweat pads, pet sheets, portable toilet members, animal excrement treatment materials, and the like.

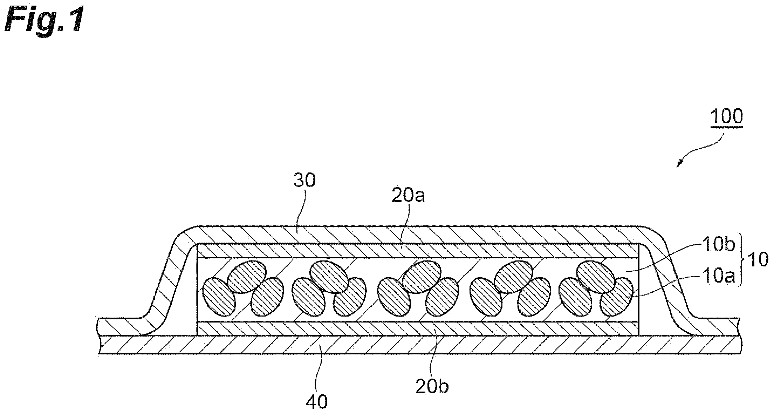

Fig. 1 is a cross-sectional view showing an example of an absorbent article. An absorbent article 100 shown in Fig. 1 includes an absorber 10, core wraps 20a and 20b, a liquid permeable sheet 30, and a liquid impermeable sheet 40. In the absorbent article 100, the liquid impermeable sheet 40, the core wrap 20b, the absorber 10, the core wrap 20a, and the liquid permeable sheet 30 are laminated in this order. In Fig. 1, there is a portion shown to be a gap between the members, but the members may be in close contact with each other without the gap.

The absorber 10 has water-absorbent resin particles 10a according to the present embodiment and a fiber layer 10b containing a fibrous substance. The water-absorbent resin particles 10a are dispersed in the fiber layer 10b.

The core wrap 20a is disposed on one surface side of the absorber 10 (an upper side of the absorber 10 in Fig. 1) in a state of being in contact with the absorber 10. The core wrap 20b is disposed on the other surface side of the absorber 10 (a lower side of the absorber 10 in Fig. 1) in a state of being in contact with the absorber 10. The absorber 10 is disposed between the core wrap 20a and the core wrap 20b. Examples of the core wraps 20a and 20b include tissues, non-woven fabrics, woven fabrics, synthetic resin films having liquid permeation holes, net-like sheets having a mesh, and the like. The core wrap 20a and the core wrap 20b each have, for example, a main surface having the same size as that of the absorber 10.

The liquid permeable sheet 30 is disposed on the outermost part on a side from which an absorption target liquid is infiltrated. The liquid permeable sheet 30 is disposed on the core wrap 20a in a state of being in contact with the core wrap 20a. Examples of the liquid permeable sheet 30 include non-woven fabrics made of synthetic resin such as polyethylene, polypropylene, polyester, and polyamide, and porous sheets. The liquid impermeable sheet 40 is disposed on the outermost part on a side opposite to the liquid permeable sheet 30, in the absorbent article 100. The liquid impermeable sheet 40 is disposed below the core wrap 20b in a state of being in contact with the core wrap 20b. Examples of the liquid impermeable sheet 40 include sheets made of synthetic resins such as polyethylene, polypropylene, and polyvinyl chloride, and sheets made of a composite material of these synthetic resins and a non-woven fabric. The liquid permeable sheet 30 and the liquid impermeable sheet 40 each have, for example, a main surface wider than the main surface of the absorber 10, and outer edges of the liquid permeable sheet 30 and the liquid impermeable sheet 40 respectively extend around the absorber 10 and the core wraps 20a and 20b.

A magnitude relationship between the absorber 10, the core wraps 20a and 20b, the liquid permeable sheet 30, and the liquid impermeable sheet 40 is not particularly limited, and it is appropriately adjusted according to usage applications and the like of the absorbent article. Furthermore, a method of retaining the shape of the absorber 10 using the core wraps 20a and 20b is not particularly limited. The absorber may be wrapped with a plurality of the core wraps as shown in Fig. 1, or the absorber may be wrapped with one core wrap.

The absorber may be adhered to the top sheet. In a case where the absorber is sandwiched or covered by the core wrap, it is preferable that at least the core wrap and the top sheet be adhered to each other, and it is more preferable that the core wrap and the absorber be adhered to each other in addition to the adhesion of the core wrap and the top sheet. Examples of methods of adhering the absorber include a method of adhering by applying a hot melt adhesive to the top sheet at predetermined intervals in a striped shape, a spiral shape, or the like in a width direction; and a method of adhering using a water-soluble binder such as starch, carboxymethyl cellulose, polyvinyl alcohol, polyvinylpyrrolidone, and other water-soluble polymers. In addition, in a case where the absorber contains heat-sealing synthetic fibers, a method of adhering by heat-sealing of the heat-sealing synthetic fibers may be adopted.

According to the present embodiment, it is possible to provide a method of absorbing a liquid using the water-absorbent resin particles, absorber, or absorbent article according to the present embodiment. The method of absorbing a liquid according to the present embodiment includes a step of bringing an absorption target liquid into contact with the water-absorbent resin particles, absorber, or absorbent article according to the present embodiment.

According to the present embodiment, it is possible to provide a method of adjusting a permeation rate (a permeation rate of a liquid) in an absorbent article, which is a method of adjusting (for example, a method of increasing) a permeation rate using the water-absorbent resin particles, the absorber, and the absorbent article according to the present embodiment (For example, an improvement method). The method of adjusting a permeation rate according to the present embodiment includes an adjustment step of adjusting a maximum gel resistance measured by the above-described procedures (1) to (3), and a water absorption amount for physiological saline under a load of 4.14 kPa. In the adjustment step, a maximum gel resistance can be adjusted to be within each of the above-described ranges (for example, 0.3 to 1.5 N). In the adjustment step, a water absorption amount for physiological saline under a load of 4.14 kPa can be adjusted to be within each of the above-described ranges (for example, 12 mL/g or more).

According to the present embodiment, it is possible to provide a method for producing water-absorbent resin particles, the method including a selection step of selecting water-absorbent resin particles based on a maximum gel resistance measured by the following procedures (1) to (3), and a water absorption amount for physiological saline under a load of 4.14 kPa. In the selection step, a maximum gel resistance can be adjusted to be within each of the above-described ranges (for example, 0.3 to 1.5 N). In the selection step, a water absorption amount for physiological saline under a load of 4.14 kPa can be adjusted to be within each of the above-described ranges (for example, 12 mL/g or more).

According to the present embodiment, it is possible to provide a method for producing an absorber by using the water-absorbent resin particles obtained by the above-mentioned method for producing water-absorbent resin particles. The method for producing an absorber according to the present embodiment includes a particle producing step of obtaining water-absorbent resin particles by the above-described method for producing water-absorbent resin particles. The method for producing an absorber according to the present embodiment may include a step of mixing the water-absorbent resin particles and a fibrous substance after the particle producing step. According to the present embodiment, it is possible to provide a method for producing an absorbent article by using the absorber obtained by the above-described method for producing an absorber. The method for producing an absorbent article according to the present embodiment includes an absorber producing step of obtaining an absorber by the above-described method for producing an absorber. The method for producing an absorbent article according to the present embodiment may include a step of obtaining an absorbent article by using the absorber and other constituent members for an absorbent article after the absorber producing step. In this step, for example, an absorbent article is obtained by laminating the absorber and other constituent members for an absorbent article with each other.

[Examples]

Hereinafter, contents of the present invention will be described in further detail using examples and comparative examples, but the present invention is not limited to the following examples.

<Production of water-absorbent resin particles>

(Example 1)

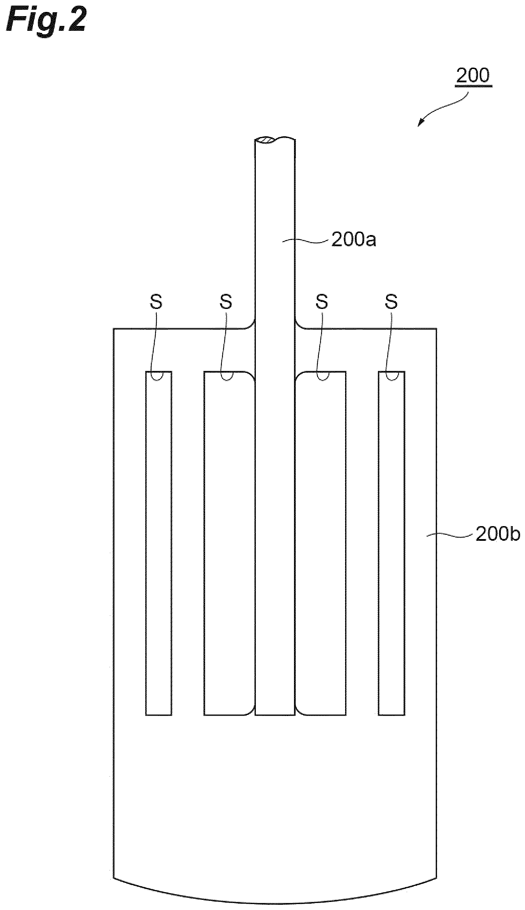

A cylindrical round-bottomed separable flask with the inner diameter of 11 cm and the internal volume of 2 L equipped with a reflux condenser, a dropping funnel, a nitrogen gas introduction tube, and a stirrer was prepared. The stirrer was equipped with a stirring blade (flat plate blade) 200 of which an outline is shown in Fig. 2. The stirring blade 200 includes a shaft 200a and a flat plate portion 200b. The flat plate portion 200b is welded to the shaft 200a and has a curved tip end. The flat plate portion 200b is formed with four slits S extending along an axial direction of the shaft 200a. The four slits S are arranged in a width direction of the flat plate portion 200b, where the width of the two slits S at the inner side is 1 cm, and the width of the two slits S at the outer side is 0.5 cm. The length of the flat plate portion 200b is about 10 cm, and the width of the flat plate portion 200b is about 6 cm. Subsequently, 293 g of n-heptane as a hydrocarbon dispersion medium was added into the above-mentioned separable flask, and 0.736 g of a maleic anhydride-modified ethylene-propylene copolymer (HI-WAX 1105A, manufactured by Mitsui Chemicals, Inc.) as a polymer-based dispersant was added thereinto. Thereby, a mixture was obtained. The dispersant was dissolved by raising the temperature to 80°C while stirring the mixture, and then the mixture was cooled to 50°C.

Subsequently, 92.0 g of an aqueous solution of 80.5% by mass acrylic acid (acrylic acid: 1.03 mole) was added into a beaker having the internal volume of 300 mL as a water-soluble ethylenically unsaturated monomer. Subsequently, while cooling the beaker from the outside, 147.7 g of an aqueous solution of 20.9% by mass sodium hydroxide was added dropwise into the beaker to perform 75 mol% of neutralization. Thereafter, 0.092 g of hydroxy ethyl cellulose (HEC AW-15F, manufactured by Sumitomo Seika Chemicals Co., Ltd.) as a thickener, 0.092 g (0.339 mmol) of 2,2'-azobis(2-amidinopropane) dihydrochloride and 0.018 g (0.067 mmol) of potassium persulfate as a water-soluble radical polymerization initiator, and 0.0046 g (0.026 mmol) of ethylene glycol diglycidyl ether as an internal cross-linking agent were added and dissolved. Thereby, a first stage aqueous liquid was prepared.

Then, the first stage aqueous liquid was added into the above-mentioned separable flask while stirring at a rotation speed of 425 rpm of the stirrer, and then stirring was performed for 10 minutes. Thereafter, 0.736 g of sucrose stearic acid ester (surfactant, Mitsubishi-Chemical Foods Corporation, RYOTO Sugar Ester S-370, HLB value: 3) was dissolved in 6.62 g of n-heptane by heating, and thereby a surfactant solution was obtained. This surfactant solution was added into the separable flask. Then, the inside of the system was sufficiently replaced with nitrogen while stirring at the rotation speed of 425 rpm of the stirrer. Thereafter, the flask was immersed in a water bath at 70°C to raise its temperature, and polymerization was performed for 60 minutes. Thereby, a first stage polymerization slurry solution was obtained.

Subsequently, 128.8 g of an aqueous solution of 80.5% by mass acrylic acid (acrylic acid: 1.44 mole) was added into another beaker having the internal volume of 500 mL as a water-soluble ethylenically unsaturated monomer. Subsequently, while cooling the beaker from the outside, 159.0 g of an aqueous solution of 27% by mass sodium hydroxide was added dropwise into the beaker to perform 75 mol% of neutralization. Thereafter, 0.129 g (0.476 mmol) of 2,2'-azobis(2-amidinopropane) dihydrochloride and 0.026 g (0.096 mmol) of potassium persulfate were added as a water-soluble radical polymerization initiator, and 0.0117 g (0.067 mmol) of ethylene glycol diglycidyl ether was added as an internal cross-linking agent. Thereafter, they were dissolved, and thereby a second stage aqueous liquid was prepared.

Subsequently, while stirring at the rotation speed of 650 rpm of the stirrer, the inside of the above-mentioned separable flask was cooled to 25°C, and then a total amount of the second stage aqueous liquid was added to the first stage polymerization slurry solution. Subsequently, after replacing the inside of the system with nitrogen for 30 minutes, the flask was immersed in a water bath at 70°C again to raise the temperature, and the polymerization reaction was performed for 60 minutes. Thereby, a second stage hydrogel-like polymer was obtained.

Under stirring, 0.589 g of an aqueous solution of 45% by mass diethylenetriaminepentaacetic acid pentasodium was added to the second stage hydrogel-like polymer. Thereafter, the temperature of the reaction solution was raised in an oil bath at 125°C, and 207.9 g of water was extracted to the outside of the system while refluxing n-heptane by azeotropic distillation of n-heptane and water. Then, 4.42 g of an aqueous solution of 2% by mass ethylene glycol diglycidyl ether (ethylene glycol diglycidyl ether: 0.507 mmol) was added into the flask as a surface cross-linking agent, and then the mixture was maintained at 83°C for 2 hours.

Thereafter, n-heptane was evaporated at 125°C, and the residue was dried to obtain polymer particles (dried product). After passing these polymer particles through a sieve having the opening of 850 μm, 0.2% by mass of amorphous silica (Tokusil NP-S manufactured by Oriental Silicas Corporation) was mixed with the polymer particles based on a total mass of the polymer particles to obtain 232.3 g of water-absorbent resin particles containing amorphous silica. A median particle diameter of the water-absorbent resin particles was 361 μm.

(Example 2)

231.0 g of water-absorbent resin particles were obtained in the same manner as that in Example 1, except that, in the hydrogel-like polymer obtained after the second stage polymerization, 224.3 g of water was extracted to the outside of the system by azeotropic distillation. The median particle diameter of the water-absorbent resin particles was 342 μm.

(Example 3)

233.0 g of water-absorbent resin particles were obtained in the same manner as that in Example 1, except that, in the hydrogel-like polymer obtained after the second stage polymerization, 229.2 g of water was extracted to the outside of the system by azeotropic distillation. The median particle diameter of the water-absorbent resin particles was 368 μm.

(Comparative Example 1)

230.8 g of water-absorbent resin particles was obtained in the same manner as in Example 1 except that, when dissolving a maleic anhydride-modified ethylene-propylene copolymer, a stirring blade having four inclined paddle blades, each having a blade diameter of 5 cm, in a two-tier manner was used as a stirrer instead of the flat plate blade; in preparation of a first stage aqueous liquid, 0.0736 g (0.272 mmol) of potassium persulfate was used as a water-soluble radical polymerization initiator, and 0.010 g (0.057 mmol) of ethylene glycol diglycidyl ether was used as an internal cross-linking agent; in preparation of a first stage polymerization slurry solution, a rotation speed of the stirrer was changed to 550 rpm; in preparation of a second stage aqueous liquid, 0.090 g (0.333 mmol) of potassium persulfate was used as a water-soluble radical polymerization initiator, and 0.012 g (0.069 mmol) of ethylene glycol diglycidyl ether was used as an internal cross-linking agent; when adding a total amount of the second stage aqueous liquid to the first stage polymerization slurry solution, a rotation speed of the stirrer was changed to 1,000 rpm; and for a hydrogel-like polymer obtained after second stage polymerization, 256.1 g of water was extracted out of the system by azeotropic distillation. A median particle diameter of the water-absorbent resin particles was 349 μm.

(Comparative Example 2)

230.6 g of water-absorbent resin particles was obtained in the same manner as in Example 1 except that, when dissolving a maleic anhydride-modified ethylene-propylene copolymer, a stirring blade having four inclined paddle blades, each having a blade diameter of 5 cm, in a two-tier manner was used as a stirrer instead of the flat plate blade; in preparation of a first stage aqueous liquid, 0.0736 g (0.272 mmol) of potassium persulfate was used as a water-soluble radical polymerization initiator, and 0.010 g (0.057 mmol) of ethylene glycol diglycidyl ether was used as an internal cross-linking agent; in preparation of a first stage polymerization slurry solution, a rotation speed of the stirrer was changed to 550 rpm; in preparation of a second stage aqueous liquid, 0.090 g (0.333 mmol) of potassium persulfate was used as a water-soluble radical polymerization initiator, and 0.012 g (0.069 mmol) of ethylene glycol diglycidyl ether was used as an internal cross-linking agent; when adding a total amount of the second stage aqueous liquid to the first stage polymerization slurry solution, a rotation speed of the stirrer was changed to 1,000 rpm; for a hydrogel-like polymer obtained after second stage polymerization, 271.4 g of water was extracted out of the system by azeotropic distillation; and 0.5% by mass of amorphous silica with respect to a mass of polymer particles was mixed with the polymer particles. A median particle diameter of the water-absorbent resin particles was 355 μm.

(Comparative Example 3)

230.8 g of water-absorbent resin particles was obtained in the same manner as in Example 1 except that, when dissolving a maleic anhydride-modified ethylene-propylene copolymer, a stirring blade having four inclined paddle blades, each having a blade diameter of 5 cm, in a two-tier manner was used as a stirrer instead of the flat plate blade; in preparation of a first stage aqueous liquid, 0.0736 g (0.272 mmol) of potassium persulfate was used as a water-soluble radical polymerization initiator, and 0.010 g (0.057 mmol) of ethylene glycol diglycidyl ether was used as an internal cross-linking agent; in preparation of a first stage polymerization slurry solution, a rotation speed of the stirrer was changed to 550 rpm; in preparation of a second stage aqueous liquid, 0.090 g (0.333 mmol) of potassium persulfate was used as a water-soluble radical polymerization initiator, and 0.0117 g (0.067 mmol) of ethylene glycol diglycidyl ether was used as an internal cross-linking agent; when adding a total amount of the second stage aqueous liquid to the first stage polymerization slurry solution, a rotation speed of the stirrer was changed to 1,000 rpm; and for a hydrogel-like polymer obtained after second stage polymerization, 278.9 g of water was extracted out of the system by azeotropic distillation. A median particle diameter of the water-absorbent resin particles was 364 $\mu$m.

(Comparative Example 4)

A cylindrical round-bottomed separable flask was prepared, which had four side wall baffles (baffle width: 7 mm), had an inner diameter of 11 cm and an internal volume of 2 L, and was equipped with a reflux condenser, a dropping funnel, a nitrogen gas introduction tube, and a stirrer (a stirring blade having four inclined paddle blades (which were surface-treated with a fluororesin), each having a blade diameter of 5 cm, in a two-tier manner). Into this flask, 451.4 g of n-heptane as a hydrocarbon dispersion medium was added, and 1.288 g of sorbitan monolaurate (NONION LP-20R, HLB value: 8.6, manufactured by NOF CORPORATION) was added as a surfactant to obtain a mixture. The sorbitan monolaurate was dissolved in n-heptane by raising the temperature to 50°C while stirring this mixture at the rotation speed of 300 rpm of the stirrer, and then the mixture was cooled to 40°C.

Subsequently, 92.0 g of an aqueous solution of 80.5% by mass acrylic acid (acrylic acid: 1.03 mole) was put into an Erlenmeyer flask having the internal volume of 500 mL. Subsequently, while cooling the flask with ice from the outside, 147.7 g of an aqueous solution of 20.9% by mass sodium hydroxide was added dropwise into the flask to perform neutralization of the acrylic acid. Thereby, an aqueous solution of partially neutralized acrylic acid was obtained. Next, 0.1012 g (0.374 mmol) of potassium persulfate as a water-soluble radical polymerization initiator was added into the aqueous solution of partially neutralized acrylic acid, and then dissolved. Thereby, a monomer aqueous solution was prepared.

After adding the above-mentioned monomer aqueous solution into the above-mentioned separable flask, the inside of the system was sufficiently replaced with nitrogen. Thereafter, while stirring at the rotation speed of 700 rpm of the stirrer, the flask was immersed in a water bath at 70°C and then maintained for 60 minutes to complete the polymerization. Thereby, a hydrogel-like polymer was obtained.

0.092 g of amorphous silica (Oriental Silicas Corporation, Tokusil NP-S) as a powdery inorganic flocculating agent was dispersed in 100 g of n-heptane, and thereby a dispersion liquid was obtained. While stirring at the rotation speed of 1,000 rpm of the stirrer, the obtained dispersion liquid was added into the produced polymer solution containing the hydrogel-like polymer, n-heptane, and the surfactant, and then mixed for 10 minutes. Thereafter, the flask containing the reaction solution was immersed in an oil bath at 125°C, and 129.0 g of water was extracted to the outside of the system while refluxing n-heptane by azeotropic distillation of n-heptane and water. Thereafter, 4.14 g of an aqueous solution of 2% by mass ethylene glycol diglycidyl ether (ethylene glycol diglycidyl ether: 0.475 mmol) was added as a surface cross-linking agent, and then the internal temperature was maintained at 83°C ± 2°C for 2 hours.

Thereafter, water and n-heptane were evaporated at 120°C, and dried until almost no evaporated product from the system was left out. Thereby, a dried product was obtained. This dried product was passed through a sieve having the opening of 850 $\mu$m to obtain 90.1 g of water-absorbent resin particles.

(Comparative Example 5)

222.1 g of water-absorbent resin particles was obtained in the same manner as in Example 1 except that, when dissolving a maleic anhydride-modified ethylene-propylene copolymer, a stirring blade having four inclined paddle blades, each having a blade diameter of 5 cm, in a two-tier manner was used as a stirrer instead of the flat plate blade; in preparation of a first stage aqueous liquid, 0.010 g (0.057 mmol) of ethylene glycol diglycidyl ether was used as an internal cross-linking agent; in preparation of a first stage polymerization slurry solution, a rotation speed of the stirrer was changed to 550 rpm; when adding a total amount of the second stage aqueous liquid to the first stage polymerization slurry solution, a rotation speed of the stirrer was changed to 1,000 rpm; and for a hydrogel-like polymer obtained after second stage polymerization, 241.8 g of water was extracted out of the system by azeotropic distillation. A median particle diameter of the water-absorbent resin particles was 327 $\mu$m.

(Comparative Example 6)

224.1 g of water-absorbent resin particles was obtained in the same manner as in Example 1 except that, when dissolving a maleic anhydride-modified ethylene-propylene copolymer, a stirring blade having four inclined paddle blades, each having a blade diameter of 5 cm, in a two-tier manner was used as a stirrer instead of the flat plate blade; in preparation of a first stage polymerization slurry solution, a rotation speed of the stirrer was changed to 550 rpm; in preparation of a second stage aqueous liquid, an internal cross-linking agent was not used; when adding a total amount of the second stage aqueous liquid to the first stage polymerization slurry solution, a rotation speed of the stirrer was changed to 1,000 rpm; and for a hydrogel-like polymer obtained after second stage polymerization, 244.0 g of water was extracted out of the system by azeotropic distillation. A median particle diameter of the water-absorbent resin particles was 364 μm.

<Median particle diameter>

The above-mentioned median particle diameter of the water-absorbent resin particles was measured by the following procedure. That is, JIS standard sieves were combined in the following order from the top: a sieve having an opening of 600 μm, a sieve having an opening of 500 μm, a sieve having an opening of 425 μm, a sieve having an opening of 300 μm, a sieve having an opening of 250 μm, a sieve having an opening of 180 μm, a sieve having an opening of 150 μm, and a saucer. 50 g of the water-absorbent resin particles was fed to the topmost sieve among the combination of the sieves, and using a Ro-Tap shaker (manufactured by Iida-seisakusho Japan Corporation), classification was performed according to JIS Z 8815 (1994). After the classification, a mass of the particles remaining on each of the sieves was calculated as a mass percentage with respect to a total amount to determine a particle diameter distribution. By integrating values on the sieves in descending order of the particle diameter with regard to the particle diameter distribution, a relationship between the opening of the sieve and the integrated value of mass percentages of the particles remaining on the sieve was plotted on a log-probability paper. The plotted points on the probability paper were connected with straight lines, and a particle diameter corresponding to 50% by mass of the integrated mass percentage was obtained as a median particle diameter.

<Water absorption amount for physiological saline (g/g)>

500 g of an aqueous solution of 0.9% by mass sodium chloride (physiological saline) was weighed into a beaker having a capacity of Wa)/2.0 500 mL, and while stirring the solution with a magnetic stirrer bar (8 mmφ × 30 mm, without a ring) at 600 r/min, 2.0 g of water-absorbent resin particles was dispersed therein such that a lump was not generated. The mixture was left to stand for 60 minutes in a stirred state to cause the water-absorbent resin particles to swell sufficiently. Thereafter, a mass Wa (g) of a standard sieve having an opening of 75 μm was measured in advance, and using this sieve, the content of the beaker was filtered. The sieve was left to stand for 30 minutes in an inclined state with an inclination angle of about 30 degrees with respect to the horizontal to separate and filter excess water. A mass Wb (g) of the sieve containing the swollen gel was measured, and a water-absorbing ability for physiological saline was obtained by the following formula.

$$\text{Water absorption amount for physiological saline} = (Wb - Wa)/2.0$$

<Water retention amount of water-absorbent resin particles>

A water retention amount (room temperature, 25°C ± 2°C) of the water-absorbent resin particles for physiological saline was measured by the following procedure. First, a cotton bag (cotton broadcloth No. 60, 100 mm in width × 200 mm in length) into which 2.0 g of the water-absorbent resin particles had been weighed was placed in a beaker having an internal volume of 500 mL. 500 g of physiological saline was poured into the cotton bag containing the water-absorbent resin particles at once so that a lump could not be produced. Thereafter, the upper part of the cotton bag was bound with a rubber band and left to stand for 30 minutes, and thereby the water-absorbent resin particles were swollen. The cotton bag after an elapse of 30 minutes was dehydrated for 1 minute using a dehydrator (manufactured by KOKUSAN Co., Ltd., product number: H-122) which had been set at a centrifugal force of 167 G. Thereafter, a mass Wa [g] of the dehydrated cotton bag containing the swollen gel was measured. By performing the same operation without addition of the water-absorbent resin particles, a mass Wb [g] of an empty cotton bag upon moisturizing was measured, and a water retention amount of the water-absorbent resin particles for physiological saline was calculated from the following formula. The results are shown in Table 1.

$$\text{Water retention amount } [g/g] = (Wa - Wb)/2.0$$

<Water absorption amount of water-absorbent resin particles under load>

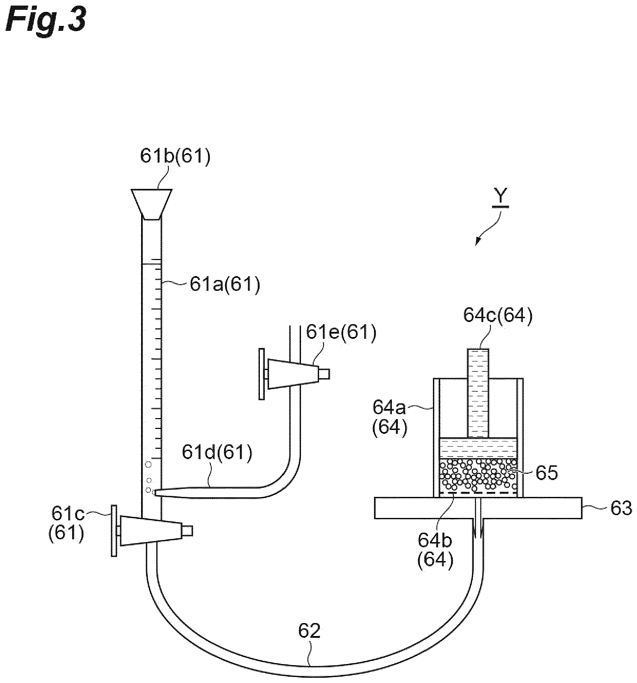

A water absorption amount (room temperature, 25°C ± 2°C) of the water-absorbent resin particles for physiological saline under a load (under pressurization) was measured using a measurement device Y shown in Fig. 3. The measurement device Y is composed of a burette unit 61, a conduit 62, a measurement table 63, and a measurement unit 64 placed on the measurement table 63. The burette unit 61 includes a burette 61a extending in a vertical direction, a rubber stopper 61b disposed at an upper end of the burette 61a, a cock 61c disposed at a lower end of the burette 61a, an air introduction tube 61d having one end extending to the burette 61a in the vicinity of the cock 61c, and a cock 61e disposed at the other end side of the air introduction tube 61d. The conduit 62 is attached between the burette unit 61 and the measurement table 63. An inner diameter of the conduit 62 is 6 mm. A hole with a diameter of 2 mm is present in a central portion of the measurement table 63, and the conduit 62 is connected to the hole. The measurement unit 64 includes a cylinder 64a (made of acrylic resin (plexiglass)), a nylon mesh 64b adhered to a bottom portion of the cylinder 64a, and a weight 64c. An inner diameter of the cylinder 64a is 20 mm. An opening of the nylon mesh 64b is 75 μm (200 mesh). Then, at the time of measurement, water-absorbent resin particles 65 which are measurement targets are uniformly sprinkled on the nylon mesh 64b. A diameter of the weight 64c is 19 mm, and a mass of the weight 64c is 119.6 g. The weight 64c can be placed on the water-absorbent resin particles 65 to apply a load of 4.14 kPa to the water-absorbent resin particles 65.

After 0.100 g of the water-absorbent resin particles 65 were put into the cylinder 64a of the measurement device Y, the weight 64c was placed thereon, and the measurement was started. The same volume of air as the physiological saline absorbed by the water-absorbent resin particles 65 was quickly and smoothly supplied to the inside of the burette 61a through the air introduction tube, and therefore a reduced amount in water level of the physiological saline inside the burette 61a was an amount of the physiological saline absorbed by the water-absorbent resin particles 65. A scale of the burette 61a was engraved from 0 mL in increments of 0.5 mL, from the top to bottom direction. A scale Va of the burette 61a before the start of water absorption and a scale Vb of the burette 61a 60 minutes after the start of water absorption were read as water levels of the physiological saline, and a water absorption amount under a load was calculated from the following formula. The results are shown in Table 1.

$$\text{Water absorption amount under load } [\text{mL/g}] = (\text{Vb} - \text{Va})/0.1$$

<Maximum gel resistance>

A small-sized stirrer (product name: Multi-Stirrer MS-101, manufacturing company: Scinics Corporation) was placed on a measurement table of EZtest (product name: EZtest, model number: EZ-SX, Shimadzu Corporation). Next, a container (product name: New Disposable Cup 100 mL) containing 58 g of physiological saline was placed on the small-sized stirrer. The container has a recessed portion having a circular cross-section, and has a first tapered portion extending along a height direction from a bottom surface, and a second tapered portion extending at a wider angle than the first tapered portion in the vicinity of the opening. A diameter of the opening end of the recessed portion is 6.4 cm. The container has a flat surface with a diameter of 4.6 cm as the bottom surface. A height of the container is 7.2 cm. A water surface of the physiological saline was located at a height of 3.1 cm from the bottom surface in the first tapered portion, and a diameter of the water surface when 58 g of the physiological saline was contained was 5.1 cm. Next, a stirrer tip (length: 3.0 cm, diameter: 8 mm, no ring) was put into the container containing the physiological saline. For the container, it is possible to use a container, in which the above-mentioned jig can be immersed at a position where a vertical distance from the upper end of the stirrer tip is 8 mm and a vertical distance below the water surface is 15 mm, when 58 g of the physiological saline was put in. The container may have a cylindrical shape not having a taper extending in the height direction as long as an inner diameter of the bottom surface is approximately 4.6 cm.

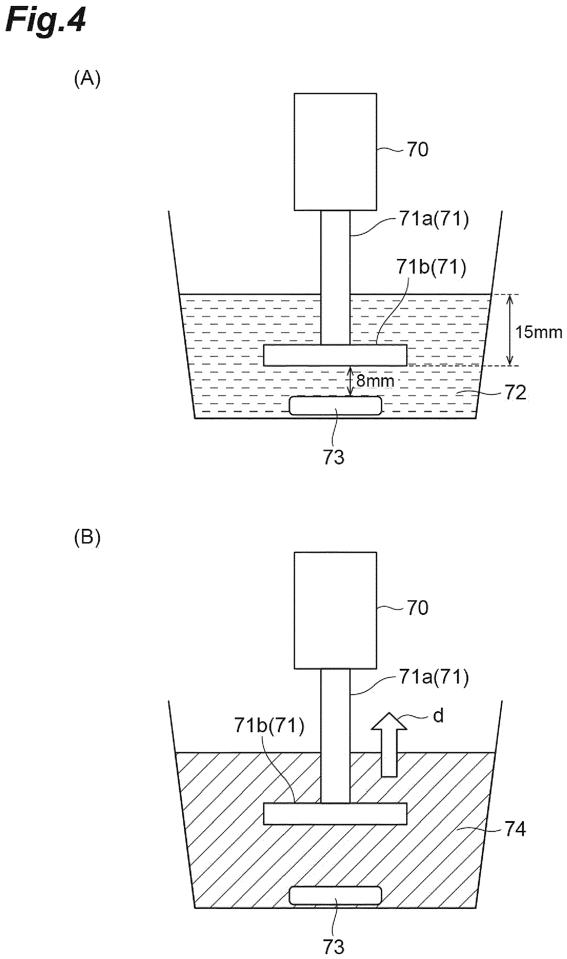

Figs. 4(A) and 4(B) show schematic views of a measurement device used for measuring a maximum gel resistance. Figs. 4(A) and 4(B) respectively show the measurement device before and after formation of a swollen gel. As shown in Figs. 4(A) and 4(B), a jig 71 is attached to a load cell 70 of the EZtest. The jig 71 includes a disk portion 71b and a rod-shaped portion 71a. The disk portion 71b has a disk shape, has flat surfaces having a diameter of 2 cm on front and back sides, and has a thickness of 5 mm. The rod-shaped portion 71a has a circular cross-section with a diameter of 5 mm, and a length of the rod-shaped portion 71a is 5.5 cm. One end of the rod-shaped portion 71a is connected to the center of a first surface which is one of the flat surfaces of the disk portion 71b, and the other end of the rod-shaped portion 71a is connected to the load cell 70. The jig 71 is disposed so that a central axis in the height direction of the container is located at the center of the disk portion 71b of the jig 71.

The jig 71 was lowered, and the movement of the jig 71 was stopped when a test force (a load in a vertical direction applied to the jig, the same shall apply hereinafter) caused by the contact of the jig 71 with the stirrer tip 73 exceeded 0.01 N. Thereafter, the jig 71 was raised by 8 mm, and this position was set as a measurement start position. As shown in Fig. 4(A), at the measurement start position, a vertical distance between a second surface (lower surface) of the disk portion of the jig 71, which is a flat surface on a side opposite to the first surface, and the stirrer tip 73 was 8 mm, and a vertical distance between the second surface (lower surface) of the disk portion of the jig 71 and a water surface of physiological saline 72 was 15 mm. The stirrer, the container, and the measurement table were fixed with tape at the measurement start position to prevent the container from moving during the measurement. A position of the jig 71 in the vertical direction was adjusted using Shimadzu software for autograph, TRAPEZIUM X (manufactured by Shimadzu Corporation).

2.0 g of water-absorbent resin particles were put into the container under stirring at 600 rpm. After confirming that the water-absorbent resin particles had swollen and vortices on a liquid surface had converged, stirring was stopped. Thereafter, the swollen particles were left to stand for 10 minutes to form a 30-fold swollen gel. As shown in Fig. 4(B), a part of the jig 71 connected to the load cell 70 is embedded in a swollen gel 74.

Ten minutes after the stirring was stopped, the jig embedded in the swollen gel was raised vertically upward (in a d direction shown in Fig. 4(B)) at a rate of 10 mm/min. A load applied to the jig when the jig was raised was measured as a test force. Measurement of a test force was performed at room temperature (temperature 25°C $\pm$ 2°C). The jig was stopped when it reached a position 17 mm higher than the measurement start position. The highest test force observed during the measurement was taken as a maximum gel resistance. The maximum gel resistance is a value automatically detected by the TRAPEZIUM X.

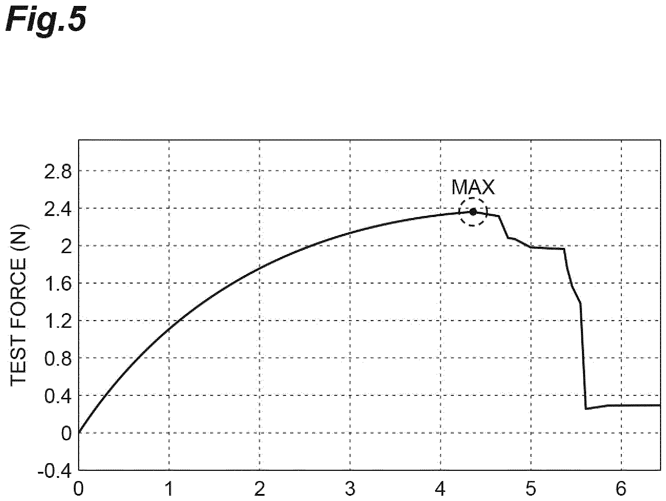

Fig. 5 is a graph showing an example of the measurement test results of the maximum gel resistance. A horizontal axis in Fig. 5 is a movement distance (displacement, unit: mm) in a vertical direction of the jig, and a vertical axis indicates a test force (unit: N). As shown in Fig. 5, a test force increases as the jig moves in the vertical direction from the measurement start position. Thereafter, the swollen gel cannot maintain its shape, and cracks, separations, and the like are generated, which decrease a test force. A maximum value of the test force (MAX value in Fig. 5) when the jig is raised in the vertical direction is obtained as a maximum gel resistance.

<Evaluation of absorbent article>

[Production of absorbent article]

10 g of the water-absorbent resin particles and 6.8 g of pulverized pulp were uniformly mixed by air papermaking using an air flow type mixer (Padformer manufactured by OTEC Co., Ltd.), and thereby a sheet-shaped absorber having a size of 40 cm $\times$ 12 cm was produced. Then, in a state where an absorber in a sheet shape was sandwiched from its upper and lower sides with two sheets of tissue paper having a basis weight of 16 g/m$^2$ and having the same size as that of the absorber, a load of 196 kPa was applied to the entire absorber for 30 seconds to press it. Thereby, a laminate was obtained. Furthermore, an air-through type porous liquid permeable sheet, which was made of polyethylene-polypropylene, had a basis weight of 22 g/m$^2$, and had the same size as that of the absorber, was disposed on the upper surface of the laminate, and thereby an absorbent article was produced.

[Measurement of permeation rate]

The absorbent article was disposed on a horizontal table in a room at a temperature of 25°C $\pm$ 2°C. Next, a liquid injection cylinder (cylinder with both ends open) having a volume of 100 mL and having an inlet with an inner diameter of 3 cm was placed at the central part of a main surface (a surface on the liquid permeable sheet side) of the absorbent article. Subsequently, 80 mL of physiological saline, which had been previously colored with a small amount of Blue No. 1 and adjusted to 25°C $\pm$ 1°C, was injected into the cylinder at one time (supplied from the vertical direction). Using a stopwatch, an absorption time from the start of the injection until the physiological saline completely disappeared from the cylinder was measured. This operation was performed twice more at intervals of 30 minutes (three times in total), and a total value of the absorption time was obtained as a permeation rate [seconds]. A permeation rate is more preferable as it becomes shorter. The results are shown in Table 1.

Table 1

| | Water-absorbent resin particles | | | | Absorbent article |
|---|---|---|---|---|---|
| | Water absorption amount [g/g] | Water retention amount [g/g] | Water absorption amount under load [mL/g] | Maximum gel resistance [N] | Permeation rate [sec.] |
| Example 1 | 51 | 34 | 28 | 1.0 | 159 |
| Example 2 | 56 | 40 | 28 | 1.0 | 164 |

(continued)

| | Water-absorbent resin particles | | | | Absorbent article |
|---|---|---|---|---|---|
| | Water absorption amount [g/g] | Water retention amount [g/g] | Water absorption amount under load [mL/g] | Maximum gel resistance [N] | Permeation rate [sec.] |
| Example 3 | 62 | 45 | 21 | 1.2 | 173 |
| Comparative Example 1 | 60 | 42 | 18 | 2.3 | 207 |
| Comparative Example 2 | 62 | 45 | 14 | 2.2 | 252 |
| Comparative Example 3 | 69 | 50 | 11 | 2.0 | 304 |
| Comparative Example 4 | 64 | 34 | 17 | 1.7 | 277 |
| Comparative Example 5 | 60 | 42 | 25 | 1.9 | 212 |
| Comparative Example 6 | 117 | 126 | 1 | 0.2 | 365 |

According to Table 1, it is confirmed that, when a maximum gel resistance is within a predetermined range, it is effective in obtaining an absorbent article having a better permeation rate.

## Reference Signs List

10: absorber, 10a, 65: water-absorbent resin particle, 10b: fiber layer, 20a, 20b: core wrap, 30: liquid permeable sheet, 40: liquid impermeable sheet, 61: burette unit, 61a: burette, 61b: rubber stopper, 61c: cock, 61d: air introduction tube, 61e: cock, 62: conduit, 63: measurement table, 64: measurement unit, 64a: cylinder, 64b: nylon mesh, 64c: weight, 70: load cell, 71: jig, 72: physiological saline, 73: stirrer tip, 74: swollen gel, 100: absorbent article, 200: stirring blade, 200a: shaft, 200b: flat plate portion, S: slit, Y: measurement device.

## Claims

1. Water-absorbent resin particles,
   wherein a maximum gel resistance measured by the following procedures (1) to (3) is 0.3 to 1.5 N, and a water absorption amount for physiological saline under a load of 4.14 kPa is 12 mL/g or more,

   (1) a jig and a cylindrical container are prepared, the jig comprising a disk portion and a rod-shaped portion, the disk portion being 5 mm in thickness and having flat surfaces with a diameter of 2 cm on front and back sides, the rod-shaped portion having a circular cross-section with a diameter of 5 mm, one end of the rod-shaped portion being connected to a center of a first surface which is one of the flat surfaces of the disk portion, the cylindrical container having a bottom surface with an inner diameter of 4.6 cm, 58 g of physiological saline being placed in the cylindrical container, and the jig and the cylindrical container are disposed so that a central axis in a height direction of the cylindrical container is located at a center of the disk portion,
   (2) a swollen gel in which the jig is embedded is formed by putting 2.0 g of the water-absorbent resin particles into the cylindrical container in a state where the jig is immersed so that a second surface of the disk portion, the second surface being a flat surface on a side opposite to the first surface, is at a position 15 mm vertically below a surface of the physiological saline, and
   (3) when the jig is raised vertically upward at a rate of 10 mm/min, a maximum value of a load applied to the jig is measured as the maximum gel resistance.

2. The water-absorbent resin particles according to claim 1, wherein the water-absorbent resin particles have a structural unit derived from at least one selected from the group consisting of (meth)acrylic acid and a salt thereof.

3. An absorber comprising the water-absorbent resin particles according to claim 1 or 2.

4. An absorbent article comprising the absorber according to claim 3.

5. The absorbent article according to claim 4, which is a diaper.

6. A method for producing water-absorbent resin particles, the method comprising:
   selecting water-absorbent resin particles based on a maximum gel resistance measured by the following procedures (1) to (3), and a water absorption amount for physiological saline under a load of 4.14 kPa,

   (1) a jig and a cylindrical container are prepared, the jig comprising a disk portion and a rod-shaped portion, the disk portion being 5 mm in thickness and having flat surfaces with a diameter of 2 cm on front and back sides, the rod-shaped portion having a circular cross-section with a diameter of 5 mm, one end of the rod-shaped portion being connected to a center of a first surface which is one of the flat surfaces of the disk portion, the cylindrical container having a bottom surface with an inner diameter of 4.6 cm, and 58 g of physiological saline being placed in the cylindrical container, and the jig and the cylindrical container are disposed so that a central axis in a height direction of the cylindrical container is located at a center of the disk portion,
   (2) a swollen gel in which the jig is embedded is formed by putting 2.0 g of the water-absorbent resin particles into the cylindrical container in a state where the jig is immersed so that a second surface of the disk portion, the second surface being a flat surface on a side opposite to the first surface, is at a position 15 mm vertically below a water surface of the physiological saline, and
   (3) when the jig is raised vertically upward at a rate of 10 mm/min, a maximum value of a load applied to the jig is measured as the maximum gel resistance.

EP 3 936 536 A1

**Fig.1**

# *Fig.2*

# Fig.3

# *Fig.4*

(A)

(B)

# Fig.5

## INTERNATIONAL SEARCH REPORT

| International application No. |
| --- |
| PCT/JP2020/009475 |

### A. CLASSIFICATION OF SUBJECT MATTER

C08F 20/06(2006.01)i; A61F 13/15(2006.01)i; A61F 13/49(2006.01)i; A61F 13/53(2006.01)i
FI: C08F20/06; A61F13/53 300; A61F13/15 329; A61F13/49

According to International Patent Classification (IPC) or to both national classification and IPC

### B. FIELDS SEARCHED

Minimum documentation searched (classification system followed by classification symbols)
C08F20/06; A61F13/15; A61F13/49; A61F13/53

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | WO 2016/006133 A1 (SUMITOMO SEIKA CHEMICALS CO., LTD.) 14.01.2016 (2016-01-14) claims 1-8, paragraphs [0001], [0123], examples 1, 2, 4-6, 8-11, table 1, comparative examples 1, 3-7, tables 1, 2 | 1-5 |
| X | WO 2016/006135 A1 (SUMITOMO SEIKA CHEMICALS CO., LTD.) 14.01.2016 (2016-01-14) examples 2-4, 7-9, comparative examples 2-7, tables 2, 3 | 1-5 |
| X | WO 2016/006129 A1 (SUMITOMO SEIKA CHEMICALS CO., LTD.) 14.01.2016 (2016-01-14) examples 1-3, comparative examples 1, 2, tables 2, 3 | 1-5 |

☐ Further documents are listed in the continuation of Box C.   ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 15 May 2020 (15.05.2020) | 26 May 2020 (26.05.2020) |

| Name and mailing address of the ISA/ Japan Patent Office 3-4-3, Kasumigaseki, Chiyoda-ku, Tokyo 100-8915, Japan | Authorized officer |
| --- | --- |
| | Telephone No. |

Form PCT/ISA/210 (second sheet) (January 2015)

INTERNATIONAL SEARCH REPORT
Information on patent family members

International application No.

PCT/JP2020/009475

| Patent Documents referred in the Report | Publication Date | Patent Family | Publication Date |
|---|---|---|---|
| WO 2016/006133 A1 | 14 Jan. 2016 | JP 2016-28117 A<br>US 2017/0107313 A1<br>claims 1-8,<br>paragraphs [0001],<br>[0125]-[0126],<br>examples 1, 2, 4-6,<br>8-11, table 1,<br>comparative examples<br>1, 3-7, tables 1, 2<br>EP 2993191 A1<br>KR 10-2016-0017649 A<br>CA 2951468 A<br>MX 2017000345 A<br>SG 112017001931< A<br>BR 112017000530 A<br>TW 201607961 A | |
| WO 2016/006135 A1 | 14 Jan. 2016 | JP 2016-28112 A<br>US 2016/0030919 A1<br>examples 2-4, 7-9,<br>comparative examples<br>2-7, tables 2, 3<br>EP 2993189 A1<br>EP 3357935 A1<br>KR 10-2016-0017648 A<br>CA 2953888 A<br>MX 2017000439 A<br>SG 11201700165S A<br>TW 201609823 A | |
| WO 2016/006129 A1 | 14 Jan. 2016 | JP 2016-28113 A<br>US 2017/0210831 A1<br>examples 1-3,<br>comparative examples<br>1, 2, tables 2, 3<br>JP 2016-28131 A<br>EP 3153528 A1<br>KR 10-2016-0142416 A<br>CA 2954027 A<br>MX 2017000342 A<br>BR 112017000533 A<br>SG 11201700162X A<br>TW 201615665 A | |

Form PCT/ISA/210 (patent family annex) (January 2015)

**EP 3 936 536 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H6345819 A

- JP H9510889 W